# FASCICULE DE BREVET EUROPEEN

(11) **EP 0 737 103 B1**
(45) Date de publication et mention de la délivrance du brevet: **12.05.1999**
(21) Numéro de dépôt: 95905677.1
(22) Date de dépôt: 28.12.1994
(51) Int. Cl.: B01J 31/20

(54) **PROCEDE DE PREPARATION D'UNE SOLUTION A BASE D'IRIDIUM ET SON UTILISATION EN TANT QUE CATALYSEUR**
VERFAHREN ZUR HERSTELLUNG EINER IRIDIUM LÖSUNG UND IHRE VERWENDUNG ALS KATALYSATOR
PROCESS FOR PRODUCING AN IRIDIUM SOLUTION AND USE OF SAME AS CATALYST.

(30) Priorité: 29.12.1993 FR 9315825
(43) Date de publication de la demande: 16.10.1996
(73) Titulaire: ACETEX CHIMIE, 92082 Paris La Défense 2 (FR)
(72) Inventeur: NOBEL, Dominique, F-30140 Salindres (FR)
(74) Mandataire: Portal, Gérard
(86) Numéro de dépôt international: FR9401542
(87) Numéro de publication internationale: WO9517963

(56) Documents cités:
- EP-A- 0 045 637
- EP-A- 0 511 126
- EP-A- 0 536 064
- WO-A-93/12063
- FR-A- 2 280 622

## Description

La présente invention a trait à la préparation d'une solution à base d'iridium, ainsi qu'à l'utilisation de celle-ci en tant que catalyseur.

L'iridium est un catalyseur bien connu et utilisé dans de nombreux types de réactions. On peut citer à titre d'exemple l'utilisation de systèmes catalytiques à base d'iridium dans des réactions de carbonylation de composés du type alcools, éthers, esters d'acides carboxyliques, dans le but de fabriquer notamment, les acides carboxyliques ou anhydrides d'acides carboxyliques. On connaît aussi des applications de l'iridium en tant que catalyseur dans des réactions d'hydroformylation d'oléfines pour obtenir des aldéhydes. Ce catalyseur peut de même être utilisé pour l'obtention d'acide acétique par isomérisation du formiate de méthyle ou encore dans la réaction de gaz à l'eau.

Le brevet FR-A-2 280 622 concerne un procédé de préparation d'acides carboxyliques en présence de compositions catalytiques à base de composés d'iridium et d'iodures. Ce document décrit différentes techniques de préparation du système catalytique mais ne propose nullement un mode de mise en solution d'un composé d'iridium difficilement soluble.

Les brevets EP-A- 536 064 et EP-A-511 126 décrivent des procédés de préparation d'acide adipique par hydrocarboxylation d'acide pentenique mettant en oeuvre un catalyseur à base d'iridium et au moins un promoteur iodé. Selon ces procédés, le catalyseur est préparé in situ dans une atmosphère nécessitant la présence de monoxyde de carbone

Dans le cas plus particulier de l'obtention d'acides carboxyliques par carbonylation, le brevet américain US 3 772 380 décrit l'utilisation d'un système catalytique, se présentant sous une forme soluble ou non dans le milieu réactionnel, à base d'iridium dont l'un des ligands est un halogène et d'un halogénure covalent (halogénure d'alkyle par exemple). L'iridium peut être introduit dans le mélange réactionnel soit directement sous la forme d'un composé comprenant l'halogène, soit sous la forme de deux composés distincts constituants des précurseurs du composé final comprenant l'iridium et l'halogène.

On constate cependant l'existence de nombreux inconvénients à procéder, à l'échelle industrielle, comme décrit dans ce brevet, et plus particulièrement dans le cadre d'une catalyse réalisée en phase homogène.

Tout d'abord, ce brevet décrit l'introduction directe d'un précurseur du catalyseur sous une forme solide, dans le milieu réactionnel. Ceci ne correspond pas à la pratique industrielle et est la cause de difficultés de mise en oeuvre. La première a trait au fait que la transformation du composé solide en un composé actif catalytiquement se traduit par une période d'initiation durant laquelle la productivité n'est pas optimale et la formation de sous-produits bien souvent augmentée. La seconde est liée au fait que le contrôle de la transformation en un composé actif soluble dans le milieu réactionnel ne peut être effectué. Par exemple, il n'est pas évident que ladite transformation soit complète et si elle ne l'est pas, que ceci n'entraîne pas une baisse d'activité et ne soit pas la cause d'une perte de catalyseur, par entraînement ou autre.

Par ailleurs, parmi les précurseurs cités se trouvent des composés contenant des éléments étrangers au système qui risquent de polluer le milieu réactionnel ou encore de perturber la conduite de la réaction.

Il est aussi à noter que dans ce brevet, la transformation en un composé actif est effectué dans les conditions de la réaction pour laquelle le catalyseur est utilisé ultérieurement, c'est-à-dire dans des conditions très dures puisque la température et la pression sont élevées.

L'un des buts de la présente invention est donc de proposer une solution à base d'iridium directement utilisable en tant que catalyseur pouvant être utilisé à une échelle industrielle, et ne présentant pas les inconvénients précités, que ce soit sur le plan de la préparation de ladite solution ou de son utilisation.

Ainsi, la présente invention permet d'obtenir une solution à base d'iridium en mettant en oeuvre un procédé simple, dans des conditions douces ne nécessitant pas notamment l'usage de pression et température élevées. De ce fait, les appareillages nécessaires dans lesquels la transformation a lieu sont des appareillages classiques et moins coûteux que des appareillages capables de résister à des hautes pressions.

L'invention permet de même la préparation d'une solution à base d'iridium ne comprenant pas d'éléments étrangers à la réaction ultérieure dans laquelle elle sera employée.

Un autre but de l'invention est d'obtenir des solutions concentrées en iridium. Ceci représente un avantage particulièrement intéressant, vu sous un angle industriel puisque cela permet de diminuer la taille des appareillages mis en oeuvre pour la préparation de la solution ou encore le nombre de campagnes de production nécessaires pour obtenir la quantité désirée en composé soluble d'iridium

Enfin, l'invention a pour objet de préparer une solution à base d'iridium directement active lorsqu'elle est utilisée en tant que catalyseur, c'est-à-dire sans période d'initiation.

Ces buts et d'autres sont atteints par la présente invention qui consiste à mettre en contact, en phase liquide, un composé carbonylé d'iridium avec de l'acide iodhydrique ou un précurseur d'un tel acide, en présence d'un solvant, sous une pression totale comprise entre 1 et 10 bar et à une température au plus égale à la température d'ébullition du solvant précité, dans les conditions de la mise en contact.

L'invention a de même pour objet l'utilisation de la solution à base d'iridium susceptible d'être obtenue en mettant en oeuvre un tel procédé, en tant que catalyseur.

Mais d'autres avantages et caractéristiques de la présente invention apparaîtront plus clairement à la lecture de la description et des exemples qui vont suivre.

Ainsi que cela a été indiqué plus haut, le procédé consiste à utiliser Ir₄(CO)₁₂ comme composé à base d'iridium

Le composé à base d'iridium est donc mis en contact avec de l'acide iodhydrique, un précurseur de cet acide, ou leur mélange.

Comme précurseur susceptible de libérer l'acide iodhydrique, on peut mentionner par exemple l'iode, les iodures d'alkyle en C₁-C₁₀ ou encore les iodures d'alcoyle en C₁-C₁₀.

L'acide iodhydrique peut être mis en oeuvre sous la forme d'une solution, ou encore sous la forme de gaz.

Selon un mode préféré de réalisation de l'invention, l'acide iodhydrique est utilisé sous la forme d'une solution, et plus particulièrement sous la forme d'une solution aqueuse. Bien que tous les degrés de dilution de l'acide conviennent à la mise en oeuvre du procédé, on préfère utiliser des solutions aqueuses présentant un titre en acide compris entre 40 et 70%.

La quantité d'acide iodhydrique mise en jeu dans le procédé selon l'invention varie dans de larges limites.

Plus particulièrement la quantité d'acide iodhydrique est telle que le nombre de moles d'acide, rapporté au nombre de moles d'iridium varie entre 1 et 20.

La mise en contact de ces deux composés est effectuée en présence d'un solvant.

Tous les solvants peuvent être utilisés dans la mesure où ils solubilisent l'acide iodhydrique ou son précurseur et le composé à base d'iridium obtenu. Toutefois, on met en oeuvre plus particulièrement, des solvants choisis parmi l'eau, les acides carboxyliques insaturés ou non, linéaires, ramifiés ou cycliques, comprenant de 1 à 10 atomes de carbone, les esters d'acides carboxyliques insaturés ou non, linéaires, ramifiés ou cycliques, comprenant 2 à 20 atomes de carbone, les alcènes ou encore les alcynes, linéaires, ramifiés ou cycliques, comprenant 2 à 20 atomes de carbone, ces composés étant pris seuls ou en mélange.

De préférence, le choix du solvant est déterminé en fonction de l'application ultérieure de la solution obtenue. Ainsi, à titre d'exemple, on peut choisir l'acide acétique et/ou l'acétate de méthyle en cas de préparation d'acide acétique. On peut utiliser l'acide adipique, l'acide 3-penténoïque, et/ou des esters correspondants, ou encore le butadiène, pour l'obtention de l'acide adipique.

D'une façon totalement surprenante et avantageuse, il a été trouvé que le composé à base l'iridium, mis en contact avec l'acide iodhydrique et/ou un précurseur, se transformait en un composé soluble, actif catalytiquement, dans des conditions très douces en température et en pression.

En effet, le procédé selon l'invention est mis en oeuvre sous une pressions totale comprise entre 1 et 10 bar.

D'après un mode préféré de réalisation de l'invention, la pression totale est de 1 à 5 bar. Selon un mode encore plus préféré, la pression totale est comprise entre 1 et 3 bar.

Par ailleurs, la réaction est menée à une température au plus égale à la température d'ébullition du solvant, dans les conditions de pression mentionnées ci-dessus.

L'atmosphère sous laquelle est mis en oeuvre le procédé de l'invention est indifférente, et ceci représente non seulement un avantage certain par rapport à ce qu'il était connu de faire, mais aussi un fait totalement inattendu.

En effet, on a trouvé que la transformation en un composé soluble de l'iridium pouvait être effectuée sous air. Or il n'était absolument pas évident pour l'homme du métier, d'une part, que ledit composé d'iridium se transforme en un composé soluble dans de telles conditions, et d'autre part, que le composé résultant soit stable. Il est rappelé ici que les procédés connus sont exclusivement mis en oeuvre sous une pression partielle élevée en monoxyde de carbone et que de telles solutions sont utilisées immédiatement après leur préparation, sinon simultanément.

On peut de même effectuer le procédé de l'invention sous un gaz rare comme l'argon ou l'hélium, sous azote ou encore sous hydrogène.

Il est précisé que travailler dans des conditions pour lesquelles on ait un mélange des gaz précités est envisageable.

Le choix de l'atmosphère, sous laquelle la transformation a lieu est fonction, d'une façon avantageuse, de l'utilisation ultérieure de la solution préparée. En effet, dans la plupart des réactions mentionnées pour lesquelles la solution précitée peut être utilisée en tant que catalyseur, une atmosphère contrôlée est nécessaire.

Par conséquent, il peut être avantageux de réaliser la réaction selon l'invention directement en utilisant un gaz souhaité pour la réaction ultérieure.

La présente invention permet d'obtenir des solutions concentrées en iridium soluble puisqu'elles peuvent contenir jusqu'à 3 % en iridium. Par ailleurs, il est important de noter que de telles solutions sont stables dans le temps, même à de telles concentrations, sans qu'il soit nécessaire de les stocker sous une atmosphère particulière, comme par exemple le monoxyde de carbone ou un gaz inerte tel que l'azote.

La mise en contact proprement dite des réactifs peut être réalisée selon toutes méthodes connues de l'homme de l'art.

Ainsi, le composé à base d'iridium peut être introduit dans l'acide iodhydrique ou un précurseur de cet acide, sachant qu'une introduction inversée ou une mise en contact simultanée des deux réactifs est possible.

Par ailleurs, l'un et/ou l'autre de ces deux composés peuvent être mis en contact directement ou bien, chacun, sous la forme d'un mélange avec l'un ou plusieurs des solvants précités.

Dans le cas où le procédé est effectué sous un gaz différent de l'air, celui-ci peut être installé avant ou pendant la mise en contact des réactifs.

Une autre possibilité envisageable, appropriée pour une utilisation immédiate de la solution à base d'iridium, est d'effectuer la mise en contact sous air. Puis, avant d'introduire la solution obtenue dans le milieu réactionnel, l'air est purgé pour le remplacer par l'atmosphère appropriée à la réaction ultérieure dans laquelle ladite solution est engagée comme catalyseur.

D'une façon classique, la mise en contact des réactifs est réalisée sous agitation.

Il est à noter que le procédé selon l'invention permet de solubiliser tout l'iridium présent. Cependant, il n'y aurait pas de problème particulier si tout l'iridium engagé ne se transformait pas en un composé soluble. En effet, dans la majeure partie des procédés industriels, le principal objectif n'est pas d'obtenir le rendement le plus élevé possible mais de trouver un compromis entre la productivité (rentabilité) et le rendement.

Ainsi, la durée de l'opération n'est pas critique et l'homme du métier est à même de la fixer, selon qu'il privilégie la rentabilité du procédé ou encore une solubilisation maximale du composé de l'iridium. A titre d'indication, cette durée varie entre une dizaine de minutes et une vingtaine d'heures.

Comme cela a été mentionné auparavant, un autre objet de l'invention a trait à l'utilisation de ladite solution à base d'iridium en tant que catalyseur.

D'une façon avantageuse, la solution obtenue par le procédé selon l'invention peut être utilisée, telle quelle pour la réaction, comme c'est le cas pour les catalyses en phase homogène.

Elle peut de même être utilisée pour préparer un catalyseur solide (supporté ou non) en appliquant les méthodes classiques.

On peut ainsi envisager sécher la solution obtenue, en présence éventuellement du support convenable pour la réaction dans laquelle le catalyseur est destiné à être utilisé, de façon à obtenir des particules à base d'iridium. Il est par ailleurs possible d'imprégner ledit support ; chacune des étapes précitées (séchage, imprégnation) étant ou non suivies de périodes de chauffage/frittage.

Cependant, selon un mode de réalisation préféré de l'invention, la solution est utilisée en tant que catalyseur ou partie d'un système catalytique pour effectuer des réactions en phase homogène.

La solution obtenue selon l'invention peut être mise directement en contact avec le mélange réactionnel ou bien être préalablement traitée pour la rendre parfaitement compatible avec ledit milieu réactionnel. Par traitement préalable, on entend notamment ajuster les teneurs en certains des composés de la solution, ou compléter la composition de la solution en y ajoutant des constituants qui ne s'y trouvaient pas, à l'issue de son procédé de préparation, ou bien encore changer l'atmosphère sous laquelle se trouve la solution.

Ladite solution peut être plus particulièrement utilisée pour effectuer des réactions de carbonylation, d'hydroformylation, d'isomérisation.

Selon un mode préféré, la présente solution est employée pour effectuer des réactions de carbonylation en présence de monoxyde de carbone, en phase liquide, en vue d'obtenir des acides carboxyliques et/ou des anhydrides d'acides carboxyliques. Il est à noter que ce type de réaction est bien connu et qu'il a fait l'objet de nombreux brevets et publications. Par conséquent, les conditions de réactions énoncées ci-dessous ne sont données qu'à titre général et ne peuvent être considérées comme limitatives.

Les réactifs mis en oeuvre pour ce genre de réaction sont choisis parmi les composés hydrocarbonés, linéaires, ramifiés ou cycliques, saturés ou non. On peut citer à titre illustratif, les alcènes ou alcynes, en C₂-C₁₀, les alcools en C₁-C₁₀, les dérivés halogénés desdits alcools, les éthers en C₂-C₂₀, les acides carboxyliques en C₃- C₁₀, comprenant au moins une insaturation, les esters d'acides carboxyliques en C₂-C₂₀ de même que les dérivés halogénés desdits esters.

D'une façon classique, le système catalytique comprend d'une part la solution à base d'iridium et d'autre part un promoteur halogéné, choisi de préférence parmi les dérivés iodés comme les iodures d'alkyles et d'alcoyles.

Habituellement, le procédé consiste à faire réagir l'un des réactifs mentionnés ci-dessus en présence du système catalytique et de monoxyde de carbone, à une température variant de 50 à 300°C et sous une pression totale comprise entre 5 et 200 bar.

La réaction est en général effectuée en présence d'un solvant qui est plus particulièrement choisi parmi les produits et/ou réactifs mis en oeuvre dans la réaction.

Selon que l'objectif de la réaction est l'obtention de l'acide carboxylique (ou l'ester correspondant) ou l'anhydride d'acide carboxylique, la réaction est mise en oeuvre dans des conditions anhydres ou non. Ainsi, dans le premier cas, le milieu réactionnel comprend en général de l'eau alors que ce ne peut être le cas lors de l'obtention d'anhydride.

Une application préférée de la solution catalytique selon l'invention consiste à effectuer la carbonylation d'alcools, ou des dérivés halogénés correspondants, en acides carboxyliques.

Dans ce dernier cas, la réaction est menée de préférence en maintenant dans le milieu réactionnel des teneurs en eau, en promoteur halogéné, en alcool, comprises entre 0 exclu et 10 %, une teneur en ester, correspondant à l'alcool et à l'acide carboxylique formé, comprise entre 2 et 40%, le reste étant constitué par ledit acide formé.

Des exemples concrets non limitatifs de l'invention vont maintenant être présentés.

### EXEMPLES

### EXEMPLE 1

Cet exemple illustre la préparation selon l'invention en utilisant l'acide acétique comme solvant.

Dans un ballon en verre, on introduit :
* 10 g de Ir₄CO₁₂ ;
* 50 g d'acide iodhydrique en solution à 57% dans l'eau ;
* 290 g d'acide acétique.

On chauffe sous agitation et sous air le mélange obtenu à reflux (≈120°C).

La durée de la réaction est de 4 heures.

On obtient une solution homogène.

On dose l'iridium dissous dans la phase liquide par spectroscopie à absorption atomique et l'on constate que 100% de l'iridium introduit est en solution.

La solution obtenue a été stockée plusieurs mois sous air sans que l'on constate de précipitation.

### EXEMPLE 2

Cet exemple illustre la préparation selon l'invention en utilisant l'acide 3-penténoïque comme solvant.

Dans un ballon en verre on introduit :
* 0,5 g de Ir₄CO₁₂ ;
* 1 g d'acide iodhydrique en solution à 57 % dans l'eau
* 38,8 g d'acide 3-penténoïque.

On chauffe sous agitation et sous air le mélange obtenu à reflux (≈150°C).

La durée de la réaction est de 2,5 heures.

On obtient une solution homogène.

On dose l'iridium dissous dans la phase liquide par spectroscopie à absorption atomique et l'on constate que 100% de l'iridium introduit est en solution.

La solution obtenue a été stockée plusieurs mois sous air sans que l'on constate de précipitation d'un composé à base d'iridium.

### EXEMPLE 3 COMPARATlF

Cet exemple est réalisé en n'utilisant pas d'acide iodhydrique.

Dans une ampoule en verre on introduit :
* 0,11 g de Ir₄CO₁₂ ;
* 7,2 g d'iodure de méthyle ;
* 20 g de méthanol ;
* 49,5 g d'acide acétique.

On chauffe sous agitation et sous air le mélange obtenu à reflux (≈120°C).

La durée de la réaction est de 4 heures.

On obtient une suspension. On filtre celle-ci et l'on dose l'iridium dissous dans la phase liquide par spectroscopie à absorption atomique. On constate que 15% de l'iridium engagé est en solution.

### EXEMPLE 4

Cet exemple a pour objet l'application de la solution catalytique obtenue dans l'exemple 1 pour la carbonylation du méthanol en acide acétique.

Dans un autoclave en Hastelloy® B2 (Haynes), on introduit :
* 2,9 g de la solution obtenue à l'exemple 1 ;
* 2,16 g d'eau ;
* 0,5 g de méthanol ;
* 8,6 g d'acétate de méthyle ;
* 2,5 g d'iodure de méthyle ;
* 54,6 g d'acide acétique.

L'autoclave est pressurisé avec 5 bar de monoxyde de carbone.

On porte la température à 190°C puis on établit, une fois la température requise atteinte, une pression totale de 30 bar, au moyen de monoxyde de carbone.

Dans ces conditions la vitesse de carbonylation du méthanol, obtenue par la mesure du débit de consommation du monoxyde de carbone, est de 4,2 mol/h.l.

Il n'a pas été constaté l'existence d'une période d'initiation de la réaction (la vitesse mentionnée ci-dessus a été atteinte immédiatement).

### EXEMPLE 5

Cet exemple a pour objet l'application de la solution catalytique obtenue dans l'exemple 2 pour la carbonylation de l'acide 3-penténoïque en acide adipique.

Dans une ampoule en verre, on introduit :
* 0,85 g de la solution obtenue à l'exemple 2 ;
* 0,87 g d'eau ;
* 9,4 g d'acide 3-penténoïque

On place l'ampoule dans un autoclave en Hastelloy® B2 (Haynes) que l'on pressurise avec 5 bar de monoxyde de carbone.

On porte la température à 185°C puis on établit, une fois la température requise atteinte, une pression totale de 20 bar, au moyen de monoxyde de carbone.

Dans ces conditions la vitesse de carbonylation de l'acide 3-penténoïque, obtenue par la mesure du débit de consommation du monoxyde de carbone, est de 8 mol/h.l.

Il n'a pas été constaté l'existence d'une période d'initiation de la réaction (la vitesse mentionnée ci-dessus a été atteinte immédiatement).

### EXEMPLE 6 COMPARATlF

Cet exemple illustre la préparation et simultanément la mise en oeuvre du catalyseur obtenu dans une réaction de carbonylation de l'acide 3-penténoïque.

Dans une ampoule en verre, on introduit :
* 10,8 mg de Ir₄CO₁₂ ;
* 21,4 mg d'acide iodhydrique en solution à 57 % dans l'eau ;
* 0,8 g d'eau ;
* 10,1 g d'acide 3-penténoïque.

On place l'ampoule dans un autoclave en Hastelloy® B2 (Haynes) que l'on pressurise avec 5 bar de monoxyde de carbone.

On porte la température à 185°C puis on établit, une fois la température requise atteinte, une pression totale de 20 bar, au moyen de monoxyde de carbone.

Dans ces conditions, on observe une période d'initiation de 30 minutes puis on mesure la vitesse de carbonylation, par mesure du débit de consommation du monoxyde de carbone. Celle-ci est de 4,4 mol/h.l.

## Revendications

1. Procédé de préparation d'une solution à base d'iridium caractérisé en ce que l'on met en contact, en phase liquide, Ir₄(CO)₁₂ avec de l'acide iodhydrique ou un précurseur d'un tel acide ou un mélange de ces produits, de telle sorte que le nombre de moles d'acide iodhydrique, rapporté au nombre de moles d'iridium, soit compris entre 1 et 20, en présence d'un solvant, sous une pression totale comprise entre 1 et 10 bar, à une température au plus égale à la température d'ébullition du solvant précité dans les conditions de la mise en contact qui est faite sous air, ou sous un gaz rare comme l'argon ou l'hélium, sous azote ou sous hydrogène, ces gaz étant utilisés seuls ou en mélange.

2. Procédé selon la revendication 1, caractérisé en ce que la mise en contact des réactifs est effectuée sous air.

3. Procédé selon l'une des revendications précédentes, caractérisé en ce que l'on effectue la mise en contact sous une pression totale comprise entre 1 et 5 bar et de préférence entre 1 et 3 bar.

4. Procédé selon l'une des revendications précédentes, caractérisé en ce que l'on utilise un solvant choisi parmi l'eau, les acides carboxyliques, linéaires, cycliques ou ramifiés, saturés ou non, comprenant 1 à 10 atomes de carbone, les esters d'acides carboxyliques saturés ou non, linéaires, cycliques ou ramifiés, comprenant 2 à 20 atomes de carbone, les alcènes, les alcynes, linéaires, cycliques ou ramifiés, comprenant 2 à 20 atomes de carbone, ou leurs mélanges.

5. Utilisation de la solution à base d'iridium susceptible d'être obtenue en mettant en oeuvre le procédé selon l'une des revendications 1 à 4, soit directement en tant que catalyseur d'une réaction en phase homogène, soit en tant que partie d'un système catalytique pour effectuer une réaction en phase homogène, soit pour préparer un catalyseur solide.

6. Utilisation selon la revendication 5, caractérisée en ce que ladite solution est utilisée pour effectuer une réaction de carbonylation, d'hydroformylation ou d'isomérisation.

7. Utilisation selon la revendication 5 ou 6, en tant que catalyseur pour les réactions de carbonylation d'alcènes, d'alcynes, linéaires, cycliques ou ramifiés, en C₂-C₁₀, les alcools, saturés ou non, linéaires, cycliques ou ramifiés, en C₁-C₁₀, de dérivés halogénés desdits alcools, d'éthers, saturés ou non, linéaires, cycliques ou ramifiés, en C₂-C₂₀, d' acides carboxyliques comprenant au moins une insaturation, linéaires, cycliques ou ramifiés, en C₃- C₁₀, d'esters d'acides carboxyliques, saturés ou non, linéaires, cycliques ou ramifiés, en C₂-C_{20,} de même que les dérivés halogénés desdits esters.

## Claims

1. A method of preparing an iridium-based solution, characterised in that Ir₄(CO)₁₂ is placed into contact, in the liquid phase, with hydroiodic acid or a precursor of such an acid, or a mixture of these products, such that the number of moles of hydroiodic acid, with respect to the number of moles of iridium, be between 1 and 20, in the presence of a solvent, under a total pressure between 1 and 10 bar, at a temperature at the most equal to the boiling point of the above-mentioned solvent under the conditions of placing into contact which is carried out under air, or under a rare gas such as argon or helium, under nitrogen or under hydrogen, these gases being used alone or as a mixture.

2. The method according to claim 1, characterised in that the reagents are placed into contact under air.

3. The method according to one of the preceding claims, characterised in that the placing into contact is made under a total pressure between 1 and 5 bar, and preferably between 1 and 3 bar.

4. The method according to one of the preceding claims, characterised in that a solvent is used which is selected from water, saturated or non-saturated, linear, cyclic or branched carboxylic acids having 1 to 10 carbon atoms, linear, cyclic or branched, saturated or non-saturated carboxylic acid esters having 2 to 20 carbon atoms, linear, cyclic or branched alkenes or alkynes having 2 to 20 carbon atoms, or mixtures thereof.

5. Use of the iridium-based solution obtainable by carrying out the method according to one of claims 1 to 4, either directly as a catalyst of a homogeneous phase reaction, or as part of a catalytic system for carrying out a homogeneous phase reaction, or for preparing a solid catalyst.

6. Use according to claim 5, characterised in that said solution is used for carrying out a carbonylation, a hydroformylation or an isomerisation reaction.

7. Use according to claim 5 or 6, as catalyst for carbonylation reactions of linear, cyclic or branched C₂-C₁₀ alkenes or alkynes, linear, cyclic or branched, saturated or non-saturated C₁-C₁₀ alcohols, halogenated derivatives of said alcohols, linear, cyclic or branched, saturated or non-saturated C₂-C₂₀ ethers, linear, cyclic or branched C₃-C₁₀ carboxylic acids having at least one unsaturation, linear, cyclic or branched, saturated or non-saturated C₂-C₂₀ carboxylic acid esters, as well as halogenated derivatives of said esters.

## Patentansprüche

1. Verfahren zur Herstellung einer Lösung auf Basis von Iridium, dadurch gekennzeichnet, daß man in flüssiger Phase Ir₄(CO)₁₂ in Kontakt bringt mit Iodwasserstoffsäure oder einem Vorläufer einer solchen Säure oder einer Mischung dieser Produkte in der Weise, daß die Anzahl der Mole Iodwasserstoffsäure, bezogen auf die Anzahl der Mole Iridium, zwischen 1 und 20 liegt, in Gegenwart eines Lösungsmittels unter einem Gesamtdruck zwischen 1 und 10 bar und bei einer Temperatur, die höchstens gleich der Siedetemperatur des obengenannten Lösungsmittels unter den Kontaktier-Bedingungen ist, d.h. an der Luft oder unter einem Edelgas wie Argon oder Helium, unter Stickstoff oder unter Wasserstoff, wobei diese Gase allein oder im Gemisch verwendet werden.

2. Verfahren nach Anspruch 1, dadurch gekennzeichnet, daß das Inkontaktbringen der Reagentien an der Luft durchgeführt wird.

3. Verfahren nach einem der vorhergehenden Ansprüche, dadurch gekennzeichnet, daß man das Inkontaktbringen unter einem Gesamtdruck zwischen 1 und 5 bar und vorzugsweise zwischen 1 und 3 bar durchführt.

4. Verfahren nach einem der vorhergehenden Ansprüche, dadurch gekennzeichnet, daß man ein Lösungsmittel verwendet, das ausgewählt wird aus der Gruppe Wasser, gesättigte oder ungesättigte lineare, cyclische oder verzweigte Carbonsäuren mit 1 bis 10 Kohlenstoffatomen, gesättigte oder ungesättigte, lineare, cyclische oder verzweigte Carbonsäureester mit 2 bis 20 Kohlenstoffatomen, lineare, cyclische oder verzweigte Alkene und Alkine mit 2 bis 20 Kohlenstoffatomen oder Mischungen davon.

5. Verwendung der Lösung auf Basis von Iridium, die durch Anwendung des Verfahrens nach einem der Ansprüche 1 bis 4 erhalten werden kann, entweder direkt als Katalysator für eine Reaktion in homogener Phase oder als Teil eines katalytischen Systems zur Durchführung einer Reaktion in homogener Phase oder zur Herstellung eines festen Katalysators.

6. Verwendung nach Anspruch 5, dadurch gekennzeichnet, daß die genannte Lösung verwendet wird zur Durchführung einer Carbonylierungs-, Hydroformylierungs- oder Isomerisierungs-Reaktion.

7. Verwendung nach Anspruch 5 oder 6 als Katalysator für Reaktionen zur Carbonylierung von linearen, cyclischen oder verzweigten C₂-C₁₀-Alkenen und -Alkinen, von gesättigten oder ungesättigten, linearen, cyclischen oder verzweigten C₂-C₁₀-Alkoholen, von halogenierten Derivaten der genannten Alkohole, von gesättigten oder ungesättigten, linearen, cyclischen oder verzweigten C₂-C₂₀-Ethern, von linearen, cyclischen oder verzweigten C₃-C₁₀-Carbonsäuren, die mindestens eine Unsättigung enthalten, von gesättigten oder ungesättigten, linearen, cyclischen oder verzweigten C₂-C₂₀-Carbonsäureestern sowie von halogenierten Derivaten der genannten Ester.
